# EUROPÄISCHE PATENTSCHRIFT

(11) **EP 0 946 295 B1**
(45) Veröffentlichungstag und Bekanntmachung des Hinweises auf die Patenterteilung: **03.12.2003**
(21) Anmeldenummer: 97943892.6
(22) Anmeldetag: 24.09.1997
(51) Int. Cl.: B01J 23/72, C07C 51/16, C07C 227/02

(54) **KATALYSATOR ZUR DEHYDROGENIERUNG VON AMINOALKOHOLEN ZU AMINOCARBONSÄUREN ODER VON ETHYLENGLYKOL(DERIVATEN) ZU OXYCARBONSÄUREN, VERFAHREN ZU SEINER HERSTELLUNG UND SEINE VERWENDUNG**
CATALYST FOR DEHYDROGENATION OF AMINO ALCOHOLS TO AMINO CARBOXYLIC ACIDS OR OF ETHYLENE GLYCOL (DERIVATIVES) TO OXYCARBOXYLIC ACIDS, METHOD FOR THEIR PRODUCTION AND THEIR USE
CATALYSEUR POUR DESHYDROGENATION D'AMINOALCOOLS EN ACIDES AMINOCARBOXYLIQUES ET DE (DERIVES D')ETHYLENEGLYCOLS EN ACIDES OXYCARBOXYLIQUES, SON PROCEDE DE PRODUCTION ET SON UTILISATION

(30) Priorität: 26.09.1996 DE 19639474
(43) Veröffentlichungstag der Anmeldung: 06.10.1999
(73) Patentinhaber: Akzo Nobel N.V., 6824 BM Arnhem (NL)
(72) Erfinder: EISENHUTH, Ludwig, D-63785 Obernburg (DE); BERGFELD, Manfred, D-63906 Erlenbach (DE)
(74) Vertreter: Fett, Günter, Dipl.-Ing.
(86) Internationale Anmeldenummer: EP9705224
(87) Internationale Veröffentlichungsnummer: WO98013140

(56) Entgegenhaltungen:
- WO-A-94/24091
- DE-A- 3 505 208
- GB-A- 2 148 287
- GB-A- 2 164 034
- PATENT ABSTRACTS OF JAPAN vol. 010, no. 269 (C-372), 12.September 1986 & JP 61 093146 A (NIPPON SHOKUBAI KAGAKU KOGYO CO LTD), 12.Mai 1986, in der Anmeldung erwähnt

## Beschreibung

Die Erfindung betrifft einen zur Dehydrogenierung von Aminoalkoholen zu Aminocarbonsäuren oder von Ethylenglykol(derivaten) zu Oxycarbonsäuren geeigneten neuen Katalysator, der Zirkonium, Kupfer und ggf. ein weiteres Metall enthält, wobei die genannten Metalle als Hydroxide gefällt, gewaschen, getrocknet, calciniert und reduziert werden.

Ferner betrifft die Erfindung ein Verfahren zur Herstellung eines zur Dehydrogenierung von Aminoalkoholen zu Aminocarbonsäuren oder von Ethylenglykol(derivaten) zu Oxycarbonsäuren einsetzbaren Katalysators, der Zirkonium, Kupfer und ggf. ein weiteres Metall enthält, wobei die genannten Metalle als Hydroxide gefällt, gewaschen, getrocknet, calciniert und reduziert werden.

Verfahren zur Herstellung eines Katalysators für die oben genannten Reaktionen wurden bereits mehrfach beschrieben. Diese Verfahren lassen sich im wesentlichen in vier Gruppen einteilen, die im folgenden beschrieben werden.

*Anker-Katalysatoren*: In WO-A1- 96/01146 wird die Herstellung von Katalysatoren mit Anker-Metallen beschrieben. Auf einem alkaliresistenten Träger wie z. B. Zirkoniumdioxid, Titandioxid oder Kohlenstoff wird ein Edelmetall wie z. B. Gold, Platin, Palladium oder Ruthenium als sogenannter Anker gebunden. Auf die Oberfläche der Anker werden Nichtedelmetalle wie z. B. Kupfer, Kobald, Nickel oder Cadmium mit einem Reduktionsmittel wie z. B. Formaldehyd abgeschieden.

*Raney-Katalysatoren*: In DE-C2- 3505208 wird Raney-Kupfer oder Raney-Nickel als Katalysator verwendet. In EP-A1- 0513396 wird als Katalysator Raney-Kupfer mit spezifizierten Werten für die BET-Oberfläche und für den Nickel-Gehalt verwendet. In EP-A1- 0506973 wird Raney-Kupfer als Katalysator verwendet, wobei während der katalytischen Dehydrogenierung dem Reaktionsgemisch Aluminium oder eine Aluminiumverbindung zugesetzt wird. In WO-A1- 94/24091 und US 5367112 wird Raney-Kupfer mit 10 bis 50000 ppm eines der Elemente Chrom, Titan, Niob, Tantal, Zirkonium, Vanadin, Molybdän, Wolfram, Kobalt, Nickel, Wismut, Zinn, Antimon, Blei, Germanium, Magnesium und deren Mischungen als Katalysator verwendet.

*Imprägniertes Zirkoniumoxid*: In JP-A1-61-93146 und GB-A1- 2148287 wird ein Katalysator beschrieben, zu dessen Herstellung Zirkoniumoxid mit einer wässrigen Lösung von Kupfernitrat imprägniert, getrocknet, 6 Stunden bei 500 °C an Luft calciniert und 6 Stunden bei 230 °C in einem Wasserstoffstrom reduziert wird.

*Simultanfällung von Zirkonium, Kupfer und ggf. von einem weiteren Metall*: In JP-A1- 61-93146, DE-C2- 3505208, EP-A1- 0506973, EP-A1- 0513396 und GB-A1- 2148287 wird ein Katalysator beschrieben, zu dessen Herstellung eine wässrige Lösung vorgelegt wird, die Zirkoniumoxychlorid und Kupfemitrat enthält. Zu dieser Lösung wird wässriges Natriumhydroxid zugesetzt, wodurch Zirkonium und Kupfer simultan als Hydroxid ausgefällt werden. Der gewaschene und getrocknete Niederschlag wird 3 Stunden an Luft bei 500 °C calciniert und 6 Stunden bei 230 °C in einem Wasserstoffstrom reduziert. Gemäß WO-A1- 94/24091 wird eine wässrige Lösung vorgelegt, die Zirkoniumoxychlorid-Octahydrat, Kupfernitrat-Trihydrat und Wismutnitrat-Pentahydrat oder Zinnnitrat enthält, und ansonsten wie bereits beschrieben verfahren.

Die Ankerkatalysatoren benötigen teure Edelmetalle, die 1 bis 50 Gewichtsprozent dieser Katalysatoren ausmachen. Katalysatoren aus Raney-Nickel sind pyrogen und erfordern daher einen erhöhten Sicherheitsaufwand bei ihrer Handhabung. Die Katalysatoren aus imprägniertem Zirkoniumoxid und aus simultan gefälltem Zirkonium und Kupfer benötigen für die katalytische Dehydrogenierung von Aminoalkohlen lange Reaktionszeiten. Beispielsweise wird in JP-A1- 61-93146 für die Umwandlung von Tetrahydroxyethylethylendiamin (THEEDA) zum Tetranatriumsalz von Ethylendiamintetraacetat ((EDTA)Na₄) mit einem Katalysator aus imprägniertem Zirkoniumoxid eine Reaktionszeit von 6,5 Stunden benötigt, um (EDTA)Na₄ in einer Ausbeute von 84,5 % herzustellen. Bei Verwendung des ebenfalls in JP-A1-61-93146 beschriebenen Katalysators aus simultan gefälltem Zirkonium und Kupfer benötigt man 6 Stunden, um aus THEEDA (EDTA)Na₄ in einer Ausbeute von 85 % herzustellen.

Daher besteht die Aufgabe der vorliegenden Erfindung darin, einen neuen Katalysator zur Dehydrogenierung von Aminoalkoholen oder von Ethylenglykol(derivaten) zur Verfügung zu stellen, der die beschriebenen Nachteile der bereits bekannten Katalysatoren zumindest vermindert.

Die weitere Aufgabe der vorliegenden Erfindung besteht darin, ein Herstellungsverfahren für einen Katalysator zur Dehydrogenierung von Aminoalkoholen oder von Ethylenglykol(derivaten) zur Verfügung zu stellen, der die beschriebenen Nachteile der bereits bekannten Katalysatorherstellungsverfahren zumindest vermindert.

Die zuerst genannte Aufgabe wird durch die Katalysatoren der Ansprüche 1 bis 10 gelöst.

Die weitere Aufgabe wird durch ein Verfahren gelöst, bei dem aus einer wässrigen Zirkoniumsalz-Lösung mit einer Base Zirkoniumhydroxid gefällt wird bis ein pH-Wert von 4 bis 10 erreicht wird, zur Zirkoniumhydroxid-Suspension die wässrige Lösung eines Kupfersalzes und ggf. eines weiteren Salzes hinzugefügt wird, durch Zugabe von weiterer Base Kupferhydroxid und ggf. das Hydroxid des im weiteren Salz enthaltenen Metalls ausgefällt wird bis ein pH-Wert von 8 bis 14 erreicht wird, die erhaltene Suspension filtriert, gewaschen, getrocknet, bei 450 bis 600 °C 2 bis 4 Stunden an Luft calciniert und schließlich bei 200 bis 250 °C in einem Wasserstoffstrom 2 bis 4 Stunden reduziert wird.

Eine bevorzugte Verfahrensweise besteht darin, daß durch Zugabe von Base Zirkoniumhydroxid gefällt wird bis ein pH-Wert von 5 bis 9 erreicht wird.

Eine weitere bevorzugte Verfahrensweise besteht darin, daß durch Zugabe von weiterer Base Kupferhydroxid und ggf. das Hydroxid des im weiteren Salz enthaltenen Metalls ausgefällt wird bis ein pH-Wert von etwa 9 bis 12 erreicht wird.

Als Base zur Hydroxidfällung können im Prinzip alle üblichen Alkali- oder Erdalkalihydroxide wie zum Beispiel Natriumhydroxid, Kaliumhydroxid, Magnesiumhydroxid oder Calciumhydroxid eingesetzt werden. Bevorzugt wird wässriges Natriumhydroxid oder Calciumhydroxid verwendet. Ferner können als Base zur Hydroxidfällung im Prinzip alle üblichen wasserlöslichen Amine wie zum Beispiel Ammoniak, Methylamin, Dimethylamin oder Trimethylamin verwendet werden, wobei eine wässrige Lösung von Ammoniak oder von Trimethylamin bevorzugt wird.

Als Zirkoniumsalz können im Prinzip alle wasserlöslichen Salze des Zirkoniums eingesetzt werden. Beispielhaft seien Zirkoniumoxychlorid, Zirkoniumsulfat, Zirkoniumoxalat, Zirkoniumnitrat, Zirkoniumacetat oder Zirkoniumperchlorat genannt. Bevorzugt werden als Zirkoniumsalz Zirkoniumoxychlorid, Zirkoniumacetat oder Zirkoniumnitrat eingesetzt.

Als Kupfersalz können im Prinzip alle wasserlöslichen organischen oder anorganischen Salze eingesetzt werden wie zum Beispiel Kupfernitrat, Kupferchlorid, Kupfersulfat oder Kupferacetat. Bevorzugt werden als Kupfersalz Kupfemitrat, Kupferchlond oder Kupferacetat eingesetzt.

Als weiteres Salz werden bevorzugt Molybdänpentachlorid oder das Nitrat oder Chlorid von Calcium, Eisen, Aluminium, Chrom, Wismut, Barium oder Magnesium eingesetzt. Die erfindungsgemäß verwendeten Salze können wasserfrei sein oder Kristallwasser enthalten. Beispiele für Salze mit Kristallwasser sind Zirkoniumoxychlorid-Octahydrat, Kupfernitrat-Trihydrat, Calciumnitrat-Tetrahydrat, Eisennitrat-Nonahydrat, Wismutnitrat-Pentahydrat und Magnesiumnitrat-Hexahydrat.

Im allgemeinen beträgt das Gewichtsverhältnis von Zirkonium zu Kupfer in den zur Herstellung des Katalysators verwendeten Salzlösungen 0,5 : 1 bis 10 : 1. Bevorzugt beträgt das Gewichtsverhältnis von Zirkonium zu Kupfer 1 : 1 bis 5 : 1.

Das Gewichtsverhältnis von Kupfer zum ggf. in der Kupfersalzlösung enthaltenen weiteren Metall beträgt im allgemeinen 250 : 0,1 bis 250 : 10, bevorzugt 250 : 1 bis 250 : 7.

Der erfindungsgemäße Katalysator wird in der durch Wasserstoff reduzierten Form verwendet. Das heißt, der Katalysator wird entweder unmittelbar nach seiner Herstellung eingesetzt, oder in der durch Wasserstoff reduzierten Form bis zu seinem Einsatz unter einer Wasserstoffatmosphäre gelagert oder erst unmittelbar vor seinem Einsatz in einem Wasserstoffstrom bei 200 bis 250 °C 2 bis 4 Stunden lang reduziert.

Der Einsatz des erfindungsgemäßen Katalysators zur Dehydrogenierung von Aminoalkoholen zu Aminocarbonsäuren oder von Ethylenglykol(derivaten) zu Oxycarbonsäuren kann als Suspension oder als Extrudat oder trägergebunden erfolgen, wobei als Träger im Prinzip alle alkaliresistenten Materialien geeignet sind.

Der erfindungsgemäße Katalysator wird zur Dehydrogenierung von Aminoalkoholen mit 1 bis 50 C-Atomen zu Aminocarbonsäuren verwendet. Beispiele für diesen Einsatz des Katalysators sind die Reaktionen von Ethanolamin zu Glycin, von N-Methylethanolamin zu Sarkosin, von THEEDA zu EDTA, von Diethanolamin zu Iminodiessigsäure, von N,N-Bis(2-hydroxyethyl)isopropylamin zu 2-Hydroxypropylaminodiessigsäure oder von Tetrakis(hydroxyethyl)1,2-propylendiamin.

Überraschenderweise wurde gefunden, daß mit dem erfindungsgemäßen Katalysator, zu dessen Reduktion im Wasserstoffstrom nur die Hälfte der Zeit benötigt wird, die der in JP-A1- 61-93146 beschriebene Katalysator braucht, die Dehydrogenierung von Aminoalkoholen zu Aminocarbonsäuren deutlich schneller verläuft, das Reaktionsprodukt in höherer Ausbeute anfällt und deutlich weniger Nebenprodukte entstehen als mit einem Katalysator, der sich lediglich durch die simultane Fällung von Zirkonium- und Kupferhydroxid vom erfindungsgemäßen Katalysator unterscheidet.

Beispielsweise führt die Dehydrogenierung von N-Methylethanolamin zu Sarkosin mit dem erfindungsgemäßen Katalysator bei dreifacher Reaktionsgeschwindigkeit zu einer um 3 % höheren Sarkosin-Ausbeute und zu weniger Nebenprodukten, z.B. zu weniger als der Hälfte an Methylamin, als mit einem Katalysator, der sich lediglich durch die simultane Fällung von Zirkonium- und Kupferhydroxid vom erfindungsgemäßen Katalysator unterscheidet.

Auch bei der Dehydrogenierung anderer Aminoalkohole zu Aminocarbonsäuren zeigt der erfindungsgemäße Katalysator ähnliche vorteilhafte Eigenschaften. Beispielsweise führt die Dehydrogenierung von THEEDA zum Tetranatruimsatz des EDTA ((EDTA)Na₄) mit dem erfindungsgemäßen, Zirkonium, Kupfer und Calcium enthaltenden Katalysator bei 1,33fach höherer Reaktionsgeschwindigkeit zu einer um 1,5 % höheren (EDTA)Na₄ - Ausbeute und zu einer deutlich geringeren Nebenproduktbildung als mit einem Katalysator, der sich lediglich durch die simultane Fällung von Zirkonium- Kupfer- und Calciumhydroxid vom erfindungsgemäßen Katalysator unterscheidet.

Es hat sich im übrigen herausgestellt, daß bei der Dehydrogenierung von Aminoalkoholen, die primäre und sekundäre Hydroxylgruppen enthalten, der erfindungsgemäße Katalysator nur an der primären Hydroxylgruppe zur Dehydrogenierung führt. Beispielsweise werden bei der Dehydrogenierung von 2-Hydroxypropanoldiethanolamin nur die an den Ethanolresten gebundenen Hydroxylgruppen zu Carbonsäuren oxidiert, während die Hydroxylgruppe am Propanolrest unverändert bleibt.

Der erfindungsgemäße Katalysator wird femer zur Dehydrogenierung von Ethylenglykol(derivaten) verwendet. Damit sind oligo- oder polymere Ethylenglykole gemeint, wie z.B. Triethylenglykol, das zum Di-Natriumsalz der 3.5. Dioxahexandicarbonsäure umgesetzt wird. Ferner sind Ethylenglykolderivate gemeint, wie z.B. 2-Methoxyethanol, das zum Na-Salz der Methoxyessigsäure dehydrogeniert wird.

Die Herstellung des erfindungsgemäßen Katalysators und seine Verwendung zu Dehydrognierungsreaktionen wird anhand der nachfolgenden Beispiele und Vergleichsbeispiele detalliert beschrieben.

### Beispiel 1:

*Herstellung des Katalysators*: Aus einer Lösung von 116,65 g Zirkoniumoxychlorid-Octahydrat (362 mmol) in 1,8 Liter Wasser wird durch Zugabe von 25%iger wässriger Natronlauge Zirkoniumhydroxid gefällt bis ein pH-Wert von 7 erreicht wird. Zur Zirkoniumhydroxid-Suspension wird eine Lösung von 18,84 g Kupfernitrat-Trihydrat (78 mmol) in 400 ml Wasser hinzugefügt und anschließend durch Zugabe von weiterer 25%iger wässriger Natronlauge Kupferhydroxid ausgefällt bis ein pH-Wert von 10,5 erreicht wird. Die Suspension wird filtriert, mit Wasser chloridfrei gewaschen und getrocknet. Der Feststoff wird 3 Stunden bei 490 °C calciniert und 3 Stunden bei 230 °C im Wasserstoffstrom reduziert und unter einer Wasserstoffatmosphäre aufbewahrt.

*Verwendung des Katalysators*: In einem eine Wasserstoffatmosphäre enthaltenden Autoklaven werden 32 g des wie im Beispiel 1 hergestellten Katalysators mit einer Lösung von 90 g Methylethanolamin (1,2 mol) und 56 g Natriumhydroxid in 135 g Wasser vorgelegt. Der Autoklav wird verschlossen, mit Wasserstoff auf einen Druck von 5 bar und anschließend auf die Reaktionstemperatur von 170 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 12 bar beträgt. Der während der Reaktion aus dem Reaktor abgelassene Wasserstoff enthält das als Nebenprodukt entstehende Methylamin, das durch Titration mit Salzsäure in einem Wäscher bestimmt wird. Das Ende der Wasserstoffentwicklung zeigt das Ende der Reaktion an. Nach beendeter Reaktion wird die Reaktionslösung über eine Filterkerze dem Reaktor entnommen und chromatographisch (HPLC) analysiert. Nach 2 Stunden Reaktionszeit wird Sarkosin in einer Ausbeute von 97 % gebildet, wobei 24 mmol Methylamin entstehen.

### Vergleichsbeispiel 1:

*Herstellung des Katalysators*: 274,2 g Zirkoniumoxychlorid-Octahydrat (851 mmol) und 44,2 g Kupfernitrat-Trihydrat werden in 2,5 Liter Wasser gelöst. Aus dieser Lösung werden durch Zugabe von 25%iger wässriger Natronlauge die Hydroxide von Zirkonium und Kupfer simultan ausgefällt bis ein pH von 10,5 erreicht wird. Anschließend wird wie in Beispiel 1 filtriert, gewaschen, getrocknet, calciniert und reduziert.

*Verwendung des Katalysators*: Der wie im Vergleichsbeispiel 1 hergestellte Katalysator wird unter den im Beispiel 1 beschriebenen Reaktionsbedingungen zur Dehydrogenierung von N-Methylethanolamin zu Sarkosin eingesetzt. Nach 6 Stunden Reaktionszeit wird Sarkosin in einer Ausbeute von 94 % gebildet, wobei 54 mmol Methylamin entstehen.

### Beispiel 2:

*Herstellung des Katalysators*: Aus einer Lösung von 116,65 g Zirkoniumoxychlorid-Octahydrat (362 mmol) in 1,8 Liter Wasser wird durch Zugabe von 25%iger wässriger Natronlauge Zirkoniumhydroxid gefällt bis ein pH-Wert von 7 erreicht wird. Zur Zirkoniumhydroxid-Suspension wird eine Lösung von 29,47 g Kupfemitrat-Trihydrat (122 mmol) und 1,16 g Calciumnitrat-Tetrahydrat (4,9 mmol) in 400 ml Wasser hinzugefügt und anschließend durch Zugabe von weiterer 25%iger wässriger Natronlauge Kupfer- und Calciumhydroxid ausgefällt bis ein pH-Wert von 10,5 erreicht wird. Die Suspension wird filtriert, mit Wasser chloridfrei gewaschen und getrocknet. Der Feststoff wird 3 Stunden bei 490 °C calciniert und 3 Stunden bei 230 °C im Wasserstoffstrom reduziert und unter einer Wasserstoffatmosphäre aufbewahrt.

*Verwendung des Katalysators*: In einem eine Wasserstoffatmosphäre enthaltenden Autoklaven werden 32 g des wie im Beispiel 2 hergestellten Katalysators mit einer Lösung von 71 g Tetrahydroxyethylethylendiamin (THEEDA) von 95,5%iger Reinheit (287 mmol reines THEEDA), 50,8 g Natriumhydroxid (1,27 mol) und 135 g Wasser vorgelegt. Der Autoklav wird verschlossen, mit Wasserstoff auf einen Druck von 1 bar und anschließend auf die Reaktionstemperatur von 195 bis 200 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 15 bar beträgt. Das Ende der Wasserstoffentwicklung zeigt das Ende der Reaktion an. Nach beendeter Reaktion wird die Reaktionslösung über eine Filterkerze dem Reaktor entnommen und chromatographisch (HPLC) analysiert. Nach 2,25 Stunden Reaktionszeit wird das Tetranatrium-Salz von Ethylendiamintetraessigsäure ((EDTA)Na₄) in einer Ausbeute von 94,5 % bezogen auf das eingesetzte THEEDA gebildet.

### Vergleichsbeispiel 2:

*Herstellung des Katalysators*: 116,65 g Zirkoniumoxychlorid-Octahydrat (362 mmol), 29,47 g Kupfemitrat-Trihydrat (122 mmol) und 1,16 g Calciumnitrat-Tetrahydrat (4,9 mmol) werden in 2,2 Liter Wasser gelöst. Aus dieser Lösung werden durch Zugabe von 25%iger wässriger Natronlauge die Hydroxide von Zirkonium, Kupfer und Calcium simultan ausgefällt bis ein pH-Wert von 10,5 erreicht wird. Anschliessend wird wie in Beispiel 2 filtriert, gewaschen, getrocknet, calciniert und reduziert.

*Verwendung des Katalysators*: Der wie im Vergleichsbeispiel 2 hergestellte Katalysator wird unter den im Beispiel 2 beschriebenen Reaktionsbedingungen zur Dehydrogenierung von EDTA zu (EDTA)Na₄ eingesetzt. Nach 3 Stunden Reaktionszeit wird (EDTA)Na₄ in einer Ausbeute von 93 % gebildet.

### Beispiel 3:

Aus einer Lösung von 116,65 g ZrOCl₂ ·8H₂O (362 mmol) in 1,8 Liter Wasser wird durch Zugabe von 25%iger wässriger NaOH Zirkoniumhydroxid gefällt bis ein pH-Wert von 7 erreicht wird. Zur Zirkoniumhydroxid-Suspension wird eine Lösung von 62,75 g Cu(NO₃)₂ ·3H₂O (260 mmol) und 0,39 g Ca(NO₃)₂ ·4H₂O (1,7 mmol) in 400 ml Wasser hinzugefügt und anschließend durch Zugabe weiterer 25%iger NaOH Kupfer- und Calciumhydroxid ausgefällt bis ein pH-Wert von 10,5 erreicht wird. Die Suspension wird filtriert, mit Wasser chloridfrei gewaschen und getrocknet. Der Feststoff wird 3 Stunden bei 490 °C calciniert. Unmittelbar vor den in den folgenden Beispielen beschriebenen Verwendungen des Katalysators wird der Feststoff 3 Stunden bei 230 °C im Wasserstoffstrom reduziert.

### Beispiel 4:

In einen eine Wasserstoffatmosphäre enthaltenden Autoklaven werden 32 g des wie in Beispiel 3 hergestellten Katalysators, 71 g THEEDA von 95,5%iger Reinheit (287 mmol reines THEEDA), 122 g Wasser und 50,8 g NaOH (1,27 mmol) eingefüllt. Der Autoklav wird verschlossen, auf einen Druck von 5 bar und anschließend auf die Reaktionstemperatur von 195 bis 200 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 15 bar beträgt. Nach 30 Minuten ist nur noch eine geringe Wasserstoffentwicklung feststellbar. Nach 2 Stunden wird praktisch kein Wasserstoff mehr gebildet, wodurch das Reaktionsende angezeigt wird. Der feste Katalysator wird unter Wasserstoffatmosphäre abfiltriert. Der Lösung wird bei 75 °C konzentrierte Salzsäure zugegeben bis ein pH-Wert von 1 erreicht ist, wobei EDTA in einer Menge von 81 g erhalten werden, die einer Ausbeute von 96,9 % bezogen auf das eingesetzte THEEDA entsprechen. Die mittels Titration mit ZnCl₂ bestimmte EDTA-Reinheit beträgt 99 %.

### Beispiel 5:

Unter den Bedingungen von Beispiel 4, jedoch mit dem Unterschied, daß die NaOH-Menge auf 55,6 g (1,39 mmol) erhöht wird, resultiert nach einer Reaktionszeit von 2 Stunden EDTA in einer Ausbeute von 97,5 % und in einer Reinheit von 99 %.

### Beispiel 6:

Unter den Bedingungen von Beispiel 4, jedoch mit dem Unterschied, daß das THEEDA nicht vorgelegt sondern innerhalb von 15 Minuten bei 190 °C zudosiert wird, resultiert nach einer Reaktionszeit von 2 Stunden (EDTA)Na₄ in einer mit HPLC bestimmten Ausbeute von 97,6 % bezogen auf das eingesetzte THEEDA.

### Beispiel 7:

In einem Autoklaven werden 100 g Diethanolamin (0,95 mol), 89,6 g NaOH (2,24 mol) in 184 g Wasser und 32 g des wie im Beispiel 3 hergestellten, jedoch nur 2 h bei 230°C im Wasserstoffstrom reduzierten Katalysators vorgelegt. Der Autoklav wird verschlossen, auf einen Druck von 1 bar und anschließend auf die Reaktionstemperatur von 160 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 10 bar beträgt. Nach beendeter Reaktion wird die Reaktionslösung unter Wasserstoffatmosphäre durch Dekantieren und Filtrieren dem Reaktor entnommen. Zum im Reaktor verbleibenden festen Katalysator werden erneut 100 g Diethanolamin, 89,6 g NaOH und 184 g Wasser zugegeben und in der eben beschriebenen Weise zur Reaktion gebracht. Auf diese Weise werden 10 Reaktionszyklen durchgeführt, wobei die einzelnen Zyklen die folgenden in Tabelle 1 dargestellten Reaktionszeiten t benötigen.

**Tabelle 1:**

| Reaktionszeit in Abhängigkeit von der Zyklenzahl | | | | | |
|---|---|---|---|---|---|
| Zyklus | 1 | 2 | 3 | 4, 5, 6, 7, 8 | 9, 10 |
| t (min) | 75 | 80 | 90 | jeweils 115 | jeweils 120 |

Nach jedem Zyklus wurde das Dinatriumsalz der Iminodiessigsäure in einer Ausbeute von > 98 % und in einer Reinheit von > 98 % erhalten (Analyse mit ¹³C-NMR und HPLC).

### Beispiel 8:

In einem Autoklaven werden 90 g N-Methylethanolamin (1,2 mol), 56 g NaOH (1,4 mol), 135 g Wasser und 32 g des Katalysators von Beispiel 7 vorgelegt. Der Autoklav wird verschlossen, auf einen Druck von 5 bar und anschließend auf die Reaktionstemperatur von 170 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 10 bar beträgt. Der während der Reaktion aus dem Reaktor abgelassene Wasserstoff enthält das als Nebenprodukt entstehende Methylamin, das wie in Beispiel 1 bestimmt wird. Nach beendeter Reaktion wird die Reaktionslösung unter Wasserstoffatmosphäre durch Dekantieren und Filtrieren dem Reaktor entnommen. Zum im Reaktor verbleibenden festen Katalysator werden erneut 90 g N-Methylethanolamin, 56 g NaOH und 135 g Wasser zugegeben und in der eben beschriebenen Weise zur Reaktion gebracht. Auf diese Weise werden 10 Reaktionszyklen durchgeführt, wobei die einzelnen Zyklen die folgenden in Tabelle 2 dargestellten Reaktionszeiten t benötigen.

**Tabelle 2:**

| Reaktionszeit in Abhängigkeit von der Zyklenzahl | | | |
|---|---|---|---|
| Zyklus | 1 | 2, 3, 4, 5, 6, 7, 8 | 9, 10 |
| t (min) | 125 | jeweils 105 | jeweils 110 |

Nach jedem Zyklus wurde das Natrium-Salz von Sarkosin in einer Ausbeute von > 98 % erhalten (Analyse mit HPLC). Pro Zyklus wurden im Abgas 12 mmol Methylamin nachgewiesen.

### Beispiel 9:

In einem Autoklaven werden 49 g N,N-Bis(2-hydroxyethyl)isopropylamin (0,3 mol), 25,2 g NaOH (0,6 mol), 135 g Wasser und 32 g des Katalysators von Beispiel 7 vorgelegt. Der Autoklav wird verschlossen, auf einen Druck von 5 bar und anschließend auf die Reaktionstemperatur von 175 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 12 bar beträgt. Nach 90 Minuten wird kein Wasserstoff mehr gebildet. Nach Filtration und Gefriertrocknung wird 2-Hydroxypropylaminodiessigsäure in einer Ausbeute von 99 % und in einer mit ¹³C-NMR analysierten Reinheit von > 99 % erhalten.

### Beispiel 10:

In einem Autoklaven werden 116,9 g N-Phenylethanolamin (0,95 mol), 41,7 g NaOH (1,04 mol) in 270 g Wasser und 32 g des wie im Beispiel 3 hergestellten, jedoch nur 2 Stunden bei 230°C im Wasserstoffstrom reduzierten Katalysators vorgelegt. Der Autoklav wird verschlossen, auf einen Druck von 5 bar und anschließend auf die Reaktionstemperatur von 195 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 14 bar beträgt. Nach 135 Minuten ist die theoretisch zu erwartende Wasserstoffmenge gebildet und die Reaktion kommt zum Stillstand. Nach Gefrier- oder Sprühtrocknung erhält man das Na-Salz von Phenylglycin als weißes Pulver in einer Ausbeute von > 97 % und in einer mit ¹³C-NMR analysierten Reinheit von > 96 %.

### Beispiel 11:

In einem Autoklaven werden 37,5 g Tetrakis(hydroxyethyl)1,2-propylendiamin (0,15 mol), 25,6 g NaOH (0,64 mol), 122 g Wasser und 32 g des Katalysators von Beispiel 7 vorgelegt. Der Autoklav wird verschlossen, auf einen Druck von 5 bar und anschließend auf die Reaktionstemperatur von 199 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 15 bar beträgt. Nach einer Reaktionszeit von 1,5 Stunden und nach Gefriertrocknung erhält man das Na-Salz der 1,2-Propylendiamintetraessigsäure als weißes Pulver in einer Ausbeute von > 96 % und in einer mit ¹³C-NMR analysierten Reinheit von > 95 %. Durch Ansäuem mit konzentrierter Salzsäure wird 1,2-Propylendiamintetraessigsäure als weißes Pulver in einer Ausbeute von 91 % erhalten.

### Beispiel 12:

In einem Autoklaven werden 91,3 g 2-Methoxyethanol (1,2 mol), 56 g NaOH (1,4 mol) in 135 g Wasser und 32 g des wie im Beispiel 3 hergestellten, jedoch nur 2 Stunden bei 230°C im Wasserstoffstrom reduzierten Katalysators vorgelegt. Der Autoklav wird verschlossen, auf einen Druck von 5 bar und anschließend auf die Reaktionstemperatur von 188 °C gebracht. Die Reaktion wird unter Rühren durchgeführt. Man läßt kontinuierlich soviel des sich während der Reaktion bildenden Wasserstoffs aus dem Autoklaven ab, daß der Druck im Autoklaven 11 bar beträgt. Das Ablassen des Wasserstoffs erfolgt über einen Kühler, dessen Kühlleistung so bemessen ist, daß mit dem Ablassen des Wasserstoffs kein nenneswerter Verlust des Alkohols verbunden ist. Nach einer Reaktionszeit von 130 Minuten und nach Gefriertrocknung erhält man das Na-Salz der Methoxyessigsäure als weißen Feststoff in einer Ausbeute von > 99 % und in einer mit ¹³C-NMR analysierten Reinheit von > 98 %.

### Beispiel 13:

In einem Autoklaven werden 90,1 g Triethylenglykol (0,6 mol), 48 g NaOH (1,2 mol) in 184 g Wasser und 32 g des wie im Beispiel 3 hergestellten, jedoch nur 2 Stunden bei 230°C im Wasserstoffstrom reduzierten Katalysators vorgelegt. Der Autoklav wird verschlossen, auf einen Druck von 5 bar und anschließend auf die Reaktionstemperatur von 180 °C gebracht. Die Reaktion wird unter Rühren durchgeführt Man läßt kontinuierlich soviel des sich während der Reaktion bildenden Wasserstoffs aus dem Autoklaven ab, daß der Druck im Autoklaven 10 bar beträgt. Nach einer Reaktionszeit von 210 Minuten und nach Gefriertrocknung erhält man das Na-Salz der 3.5.Dioxahexandicarbonsäure als weißen Feststoff in einer Ausbeute von > 99 % und in einer mit ¹³C-NMR analysierten Reinheit von > 98 %.

### Beispiel 14:

*Herstellung des Katalysators:* Der Katalysator wurde wie in Beispiel 3 hergestellt mit dem Unterschied, daß bei der Fällung des Zirkoniumhydroxids durch Zugabe von NaOH anstelle eines pH-Wertes von 7 ein pH-Wert von 5 eingestellt wurde.

*Verwendung des Katalysators*: Der Katalysator wurde wie im Beispiel 4 verwendet mit dem Unterschied, daß die eingesetzte Wassermenge auf 183 g erhöht wurde. Nach einer Reaktionszeit von 120 Minuten wurde das Tetranatrium-Salz von EDTA in einer mit HPLC bestimmten Ausbeute von 93,4 % erhalten.

### Beispiel 15

Herstellung und Verwendung des Katalysators erfolgten wie im Beispiel 14 mit dem Unterschied, daß bei der Fällung des Zirkoniumhydroxids anstelle eines pH-Wertes von 5 ein pH-Wert von 9 eingestellt wurde. Nach einer Reaktionszeit von 120 Minuten wurde das Tetranatrium-Salz von EDTA in einer mit HPLC bestimmten Ausbeute von 93,8 % erhalten.

## Patentansprüche

1. Katalysator zur Dehydrogenierung von Aminoalkoholen zu Aminocarbonsäuren oder von Ethylenglykol(derivaten) zu Oxycarbonsäuren, der Zirkonium, Kupfer und ggf. ein weiteres Metall enthält, wobei die genannten Metalle als Hydroxide gefällt, gewaschen, getrocknet, calciniert und reduziert werden, dadurch herstellbar, daß aus einer wässrigen Zirkoniumsalz-Lösung mit einer Base Zirkoniumhydroxid gefällt wird bis ein pH-Wert von 4 bis 10 erreicht wird, zur Zirkoniumhydroxid-Suspension die wässrige Lösung eines Kupfersalzes und ggf. eines weiteren Salzes hinzugefügt wird, durch Zugabe von weiterer Base Kupferhydroxid und ggf. das Hydroxid des im weiteren Salz enthaltenen Metalls ausgefällt wird bis ein pH-Wert von 8 bis 14 erreicht wird, die erhaltene Suspension filtriert, gewaschen, getrocknet, bei 450 bis 600 °C 2 bis 4 Stunden an Luft calciniert und schließlich bei 200 bis 250 °C in einem Wasserstoffstrom 2 bis 4 Stunden reduziert wird.

2. Katalysator gemäß Anspruch 1, **dadurch gekennzeichnet, daß** aus einer wässrigen Zirkoniumsalz-Lösung mit einer Base Zirkoniumhydroxid gefällt wird, bis ein pH-Wert von 5 bis 9 erreicht wird.

3. Katalysator nach Anspruch 1 oder 2, dadurch herstellbar, daß durch Zugabe von weiterer Base Kupferhydroxid und ggf. das Hydroxid des im weiteren Salz enthaltenen Metalls ausgefällt wird bis ein pH-Wert von 9 bis 12 erreicht wird.

4. Katalysator nach einem oder mehreren der Ansprüche 1 bis 3, dadurch herstellbar, daß die Base eine wässrige Lösung eines Alkali- oder Erdalkalihydroxids oder eines Amins ist.

5. Katalysator nach Anspruch 4, dadurch herstellbar, daß das Alkalihydroxid Natriumhydroxid, das Erdalkalihydroxid Calciumhydroxid und das Amin Ammoniak oder Trimethylamin ist.

6. Katalysator nach einem oder mehreren der Ansprüche 1 bis 5, dadurch herstellbar, daß das Zirkoniumsalz Zirkoniumoxychlorid, Zirkoniumsulfat, Zirkoniumoxalat, Zirkoniumnitrat, Zirkoniumacetat oder Zirkoniumperchlorat ist, das Kupfersalz Kupfernitrat, Kupferchlorid, Kupfersulfat oder Kupferacetat ist und das weitere Salz Molybdänpentachlorid oder das Nitrat oder Chlorid von Calcium, Eisen, Aluminium, Chrom, Wismut, Barium oder Magnesium ist, wobei die Salze Kristallwasser enthalten oder wasserfrei sind.

7. Katalysator nach einem oder mehreren der Ansprüche 1 bis 6, dadurch herstellbar, daß das Gewichtsverhältnis von Zirkonium zu Kupfer 0,5 : 1 bis 10 : 1 beträgt.

8. Katalysator nach Anspruch 7, dadurch herstellbar, daß das Gewichtsverhältnis von Zirkonium zu Kupfer 1 : 1 bis 5 : 1 ist.

9. Katalysator nach einem oder mehreren der Ansprüche 1 bis 8, dadurch herstellbar, daß das Gewichtsverhältnis von Kupfer zum weiteren Metall 250 : 0,1 bis 250 : 10 beträgt.

10. Katalysator nach Anspruch 9, dadurch herstellbar, daß das Gewichtsverhältnis von Kupfer zum weiteren Metall 250 : 1 bis 250 : 7 beträgt.

11. Verfahren zur Herstellung eines zur Dehydrogenierung von Aminoalkoholen zu Aminocarbonsäuren oder von Ethylenglykol(derivaten) zu Oxycarbonsäuren einsetzbaren Katalysators, der Zirkonium, Kupfer und ggf. ein weiteres Metall enthält, wobei die genannten Metalle als Hydroxide gefällt, gewaschen, getrocknet, calciniert und reduziert werden, **dadurch gekennzeichnet, daß** aus einer wässrigen Zirkoniumsalz-Lösung mit einer Base Zirkoniumhydroxid gefällt wird bis ein pH-Wert von 4 bis 10 erreicht wird, zur Zirkoniumhydroxid-Suspension die wässrige Lösung eines Kupfersalzes und ggf. eines weiteren Salzes hinzugefügt wird, durch Zugabe von weiterer Base Kupferhydroxid und ggf. das Hydroxid des im weiteren Salz enthaltenen Metalls ausgefällt wird bis ein pH-Wert von 8 bis 14 erreicht wird, die erhaltene Suspension filtriert, gewaschen, getrocknet, bei 450 bis 600 °C 2 bis 4 Stunden an Luft calciniert und schließlich bei 200 bis 250 °C in einem Wasserstoffstrom 2 bis 4 Stunden reduziert wird.

12. Verfahren gemäß Anspruch 11, **dadurch gekennzeichnet, daß** aus einer wässrigen Zirkoniumsalz-Lösung mit einer Base Zirkoniumhydroxid gefällt wird, bis ein pH-Wert von 5 bis 9 erreicht wird.

13. Verfahren nach Anspruch 11 oder 12, **dadurch gekennzeichnet, daß** durch Zugabe von weiterer Base Kupferhydroxid und ggf. das Hydroxid des im weiteren Salz enthaltenen Metalls ausgefällt wird bis ein pH-Wert von 9 bis 12 erreicht wird.

14. Verfahren nach einem oder mehrerer der Ansprüche 11 bis 13, **dadurch gekennzeichnet, daß** die Base eine wässrige Lösung eines Alkali- oder Erdalkalihydroxids oder eines Amins ist.

15. Verfahren nach Anspruch 14, **dadurch gekennzeichnet, daß** das Alkali-hydroxid Natriumhydroxid, das Erdalkalihydroxid Calciumhydroxid und das Amin Ammoniak oder Trimethylamin ist.

16. Verfahren nach einem oder mehreren der Ansprüche 11 bis 15, **dadurch gekennzeichnet, daß** das Zirkoniumsalz Zirkoniumoxychlorid, Zirkoniumsulfat, Zirkoniumoxalat, Zirkoniumnitrat, Zirkoniumacetat oder Zirkoniumperchlorat ist, das Kupfersalz Kupfemitrat, Kupferchlorid, Kupfersulfat oder Kupferacetat ist und das weitere Salz Molybdänpentachlorid oder das Nitrat oder Chlorid von Calcium, Eisen, Aluminium, Chrom, Wismut, Barium oder Magnesium ist, wobei die Salze entweder Kristallwasser enthalten oder kristallwasserfrei sind.

17. Verfahren nach einem oder mehreren der Ansprüche 11 bis 16, **dadurch gekennzeichnet, daß** das Gewichtsverhältnis von Zirkonium zu Kupfer 0,5 : 1 bis 10 : 1 beträgt.

18. Verfahren nach Anspruch 17, **dadurch gekennzeichnet, daß** das Gewichtsverhältnis von Zirkonium zu Kupfer 1 : 1 bis 5 : 1 ist.

19. Verfahren nach einem oder mehreren der Ansprüche 11 bis 18, **dadurch gekennzeichnet, daß** das Gewichtsverhältnis von Kupfer zum weiteren Metall 250 : 0,1 bis 250 : 10 beträgt.

20. Verfahren nach Anspruch 19, **dadurch gekennzeichnet, daß** das Gewichtsverhältnis von Kupfer zum weiteren Metall 250 : 1 bis 250 : 7 beträgt.

21. Verwendung des nach einem oder mehreren der Ansprüche 11 bis 20 hergestellten Katalysators in der durch Wasserstoff reduzierten Form zur Dehydrogenierung von Aminoalkoholen zu Aminocarbonsäuren.

22. Verwendung des nach einem oder mehreren der Ansprüche 11 bis 20 hergestellten Katalysators in der durch Wasserstoff reduzierten Form zur Dehydrogenierung von Ethylenglykol(derivaten) zu Oxycarbonsäuren.

## Claims

1. Catalyst for dehydrogenating amino alcohols to aminocarboxylic acids or ethylene glycol (derivatives) to oxycarboxylic acids, said catalyst containing zirconium, copper and possibly an additional metal, whereby the cited metals are precipitated as hydroxides, washed, dried, calcined, and reduced, preparable in that zirconium hydroxide is precipitated from an aqueous zirconium salt solution using a base until a pH of 4 to 10 is attained, an aqueous solution of a copper salt and optionally of an additional salt is added to the zirconium hydroxide suspension, and, by adding further base, copper hydroxide and possibly the hydroxide of the metal contained in the additional salt is precipitated until a pH of 8 to 14 is attained, the suspension obtained is filtered, washed, dried, calcined in air at 450 to 600°C for 2 to 4 hours and finally reduced at 200 to 250°C in a hydrogen stream for 2 to 4 hours.

2. Catalyst in accordance with Claim 1, **characterized in that** zirconium hydroxide is precipitated from an aqueous zirconium salt solution using a base until a pH of 5 to 9 is attained.

3. Catalyst in accordance with Claim 1 or 2, preparable in that by adding further base copper hydroxide and optionally the hydroxide of the metal contained in the additional salt is precipitated until a pH of 9 to 12 is attained.

4. Catalyst in accordance with one or more of Claims 1 to 3, preparable in that the base is an aqueous solution of an alkaline or alkaline-earth hydroxide or of an amine.

5. Catalyst in accordance with Claim 4, preparable in that the alkaline hydroxide is sodium hydroxide, the alkaline-earth hydroxide is calcium hydroxide, and the amine is ammonia or trimethylamine.

6. Catalyst in accordance with one or more of Claims 1 to 5, preparable in that the zirconium salt is zirconium oxychloride, zirconium sulfate, zirconium oxalate, zirconium nitrate, zirconium acetate, or zirconium perchlorate, the copper salt is copper nitrate, copper chloride, copper sulfate, or copper acetate, and the additional salt is molybdenum pentachloride or the nitrate or chloride of calcium, iron, aluminum, chromium, bismuth, barium, or magnesium, whereby the salts contain water of crystallization or are anhydrous.

7. Catalyst in accordance with one or more of Claims 1 to 6, preparable in that the weight ratio of zirconium to copper is 0.5:1 to 10:1.

8. Catalyst in accordance with Claim 7, preparable in that the weight ratio of zirconium to copper is 1:1 to 5:1.

9. Catalyst in accordance with one or more of Claims 1 to 8, preparable in that the weight ratio of copper to the additional metal is 250:0.1 to 250:10.

10. Catalyst in accordance with Claim 9, preparable in that the weight ratio of copper to the additional metal is 250:1 to 250:7.

11. Process for preparing a catalyst suitable for dehydrogenating amino alcohols to aminocarboxylic acids or ethylene glycol (derivatives) to oxycarboxylic acids, said catalyst containing zirconium, copper and possibly an additional metal, whereby the cited metals are precipitated as hydroxides, washed, dried, calcined, and reduced, **characterized in that** zirconium hydroxide is precipitated from an aqueous zirconium salt solution using a base until a pH of 4 to 10 is attained, the aqueous solution of a copper salt and possibly of an additional salt is added to the zirconium hydroxide suspension, and, by adding further base, copper hydroxide and optionally the hydroxide of the metal contained in the additional salt is precipitated until a pH of 8 to 14 is attained, the suspension obtained is filtered, washed, dried, calcined in air at 450 to 600°C for 2 to 4 hours and finally reduced at 200 to 250°C in a hydrogen stream for 2 to 4 hours.

12. Process in accordance with Claim 11, **characterized in that** zirconium hydroxide is precipitated from an aqueous zirconium salt solution using a base until a pH of 5 to 9 is attained.

13. Process in accordance with Claim 11 or 12, **characterized in that** by adding further base copper hydroxide and optionally the hydroxide of the metal contained in the additional salt is precipitated until a pH of 9 to 12 is attained.

14. Process in accordance with one or more of Claims 11 to 13, **characterized in that** the base is an aqueous solution of an alkaline or alkaline-earth hydroxide or of an amine.

15. Process in accordance with Claim 14, **characterized in that** the alkaline hydroxide is sodium hydroxide, the alkaline-earth hydroxide is calcium hydroxide, and the amine is ammonia or trimethylamine.

16. Process in accordance with one or more of Claims 11 to 15, **characterized in that** the zirconium salt is zirconium oxychloride, zirconium sulfate, zirconium oxalate, zirconium nitrate, zirconium acetate, or zirconium perchlorate, the copper salt is copper nitrate, copper chloride, copper sulfate, or copper acetate, and the additional salt is molybdenum pentachloride or the nitrate or chloride of calcium, iron, aluminum, chromium, bismuth, barium, or magnesium, whereby the salts either contain water of crystallization or do not contain water of crystallization.

17. Process in accordance with one or more of Claims 11 to 16, **characterized in that** the weight ratio of zirconium to copper is 0.5:1 to 10:1.

18. Process in accordance with Claim 17, **characterized in that** the weight ratio of zirconium to copper is 1:1 to 5:1.

19. Process in accordance with one or more of Claims 11 to 18, **characterized in that** the weight ratio of copper to the additional metal is 250:0.1 to 250:10.

20. Process in accordance with Claim 19, **characterized in that** the weight ratio of copper to the additional metal is 250:1 to 250:7.

21. Application of the catalyst prepared in accordance with one or more of Claims 11 to 20 in the form reduced by hydrogen for dehydrogenating amino alcohols to aminocarboxylic acids.

22. Application of the catalyst prepared in accordance with one or more of Claims 11 to 20 in the form reduced by hydrogen for dehydrogenating ethylene glycol (derivatives) to oxycarboxylic acids.

## Revendications

1. Catalyseur de déshydrogénation d'aminoalcools en acides amino-carboxyliques ou (des dérivés) d'éthylène glycols en oxacides carboxyliques, qui contient du zirconium, du cuivre et le cas échéant un autre métal, dans lequel lesdits métaux ont été précipités sous forme d'hydroxydes, lavés, séchés, calcinés et réduits, **caractérisé en ce qu'**il est fabriqué par précipitation d'hydroxyde de zirconium à partir d'une solution aqueuse de sel de zirconium avec une base et obtention d'un pH ayant une valeur de 4 à 10, par ajout à la suspension d'hydroxyde de zirconium de la solution aqueuse d'un sel de cuivre et le cas échéant d'un autre sel, par précipitation d'hydroxyde de cuivre et le cas échéant d'hydroxyde du métal contenu dans l'autre sel, par rajout de plus de base et obtention d'un pH ayant une valeur de 8 à 14, par filtration, lavage, séchage, calcination à l'air à une température de 450 °C à 600 °C pendant 2 à 4 heures et finalement réduction sous un flux d'hydrogène à une température de 200 °C à 250 °C pendant 2 à 4 heures de la suspension obtenue.

2. Catalyseur selon la revendication 1, **caractérisé en ce que** de l'hydroxyde de zirconium est précipité à partir d'une solution aqueuse de sel de zirconium avec une base, et **en ce qu'**un pH ayant une valeur de 5 à 9 est atteint.

3. Catalyseur selon la revendication 1 ou 2, **caractérisé en ce qu'**il peut être fabriqué par précipitation d'hydroxyde de cuivre, et le cas échéant d'hydroxyde du métal contenu dans un autre sel, par ajout de plus de base, jusqu'à ce qu'un pH ayant une valeur de 9 à 12 soit obtenu.

4. Catalyseur selon l'une ou plusieurs des revendications 1 à 3, **caractérisé en ce que** la base est une solution aqueuse d'un hydroxyde de métal alcalin ou de métal alcalino-terreux, ou est une amine.

5. Catalyseur selon la revendication 4, **caractérisé en ce que** l'hydroxyde de métal alcalin est de l'hydroxyde de sodium, l'hydroxyde de métal alcalino-terreux est de l'hydroxyde de calcium.et l'amine est de l'ammoniaque ou de la triméthylamine.

6. Catalyseur selon l'une ou plusieurs des revendications 1 à 5, **caractérisé en ce que** le sel de zirconium est de l'oxychlorure de zirconium, du sulfate de zirconium, de l'oxalate de zirconium, du nitrate de zirconium, de l'acétate de zirconium ou du perchlorate de zirconium, le sel de cuivre est du nitrate de cuivre, du chlorure de cuivre, du sulfate de cuivre ou de l'acétate de cuivre et l'autre sel est du pentachlorure de molybdène ou le nitrate ou le chlorure de calcium, de fer, d'aluminium, de chrome, de bismuth, de baryum ou de magnésium, dans lequel les sels contiennent de l'eau de cristallisation ou ne contiennent pas d'eau.

7. Catalyseur selon l'une ou plusieurs des revendications 1 à 6, **caractérisé en ce que** le rapport pondéral de zirconium/cuivre est de 0,5/1 à 10/1.

8. Catalyseur selon la revendication 7, **caractérisé en ce que** le rapport pondéral de zirconium/cuivre est de 1/1 à 5/1.

9. Catalyseur selon l'une ou plusieurs des revendications 1 à 8, **caractérisé en ce que** le rapport pondéral de cuivre/autre métal est de 250/0,1 à 250/10.

10. Catalyseur selon la revendication 9, **caractérisé en ce que** le rapport pondéral de cuivre/autre métal est de 250/1 à 250/7.

11. Procédé de fabrication d'un catalyseur utilisable pour la déshydrogénation d'aminoalcools en acides amino-carboxyliques ou (des dérivés) d'éthylène glycols en oxacides carboxyliques, qui contient du zirconium, du cuivre et le cas échéant un autre métal, dans lequel lesdits métaux ont été précipités sous forme d'hydroxydes, lavés, séchés, calcinés et réduits, **caractérisé en ce que** de l'hydroxyde de zirconium est précipité à partir d'une solution aqueuse de sel de zirconium avec une base et jusqu'à ce qu'une valeur de pH de 4 à 10 soit obtenue, la solution aqueuse d'un sel de cuivre et le cas échéant d'un autre sel est ajoutée à la suspension d'hydroxyde de zirconium, l'hydroxyde de cuivre et le cas échéant l'hydroxyde du métal contenu dans l'autre sel est précipité par ajout de plus de base et jusqu'à ce qu'une valeur de pH de 8 à 14 soit obtenue, la suspension obtenue est filtrée, lavée, séchée, calcinée à l'air à une température de 450 °C à 600 °C pendant 2 à 4 heures et finalement réduite sous un flux d'hydrogène à une température de 200 °C à 250 °C pendant 2 à 4 heures.

12. Procédé selon la revendication 11, **caractérisé en ce que** de l'hydroxyde de zirconium est précipité à partir d'une solution aqueuse de sel de zirconium par ajout d'une base et jusqu'à ce qu'une valeur de pH de 5 à 9 soit obtenue.

13. Procédé selon la revendication 11 ou 12, **caractérisé en ce que** l'hydroxyde de cuivre et le cas échéant l'hydroxyde du métal de l'autre sel est précipité par ajout de plus de base et jusqu' à ce qu' une valeur de pH de 9 à 12 soit obtenue.

14. Procédé selon l'une ou plusieurs des revendications 11 à 13, **caractérisé en ce que** la base est une solution aqueuse d'un hydroxyde de métal alcalin ou de métal alcalino-terreux, ou une amine.

15. Procédé selon la revendication 14, **caractérisé en ce que** l'hydroxyde de métal alcalin est de l'hydroxyde de sodium, l'hydroxyde de métal alcalino-terreux est de l'hydroxyde de calcium et l'amine est de l' ammoniaque ou de la triméthylamine.

16. Procédé selon l'une ou plusieurs des revendications 11 à 15, **caractérisé en ce que** le sel de zirconium est de l'oxychlorure de zirconium, du sulfate de zirconium, de l'oxalate de zirconium, du nitrate de zirconium, de l'acétate de zirconium ou du perchlorate de zirconium, le sel de cuivre est du nitrate de cuivre, du chlorure de cuivre, du sulfate de cuivre ou de l'acétate de cuivre et l'autre sel est du pentachlorure de molybdène ou du nitrate ou du chlorure de calcium, de fer, d'aluminium, de chrome, de bismuth, de baryum ou de magnésium, dans lequel les sels contiennent de l'eau de cristallisation ou ne contiennent pas d'eau.

17. Procédé selon l'une ou plusieurs des revendications 11 à 16, **caractérisé en ce que** le rapport pondéral de zirconium/cuivre est de 0,5/1 à 10/1.

18. Procédé selon la revendication 17, **caractérisé en ce que** le rapport pondéral de zirconium/cuivre est de 1/1 à 5/1.

19. Procédé selon l'une ou plusieurs des revendications 11 à 18, **caractérisé en ce que** le rapport pondéral de cuivre/autre métal est de 250/0,1 à 250/10.

20. Procédé selon la revendication 19, **caractérisé en ce que** le rapport pondéral de cuivre/autre métal est de 250/1 à 250/7.

21. Utilisation du catalyseur fabriqué selon l'une ou plusieurs des revendications 11 à 20 sous la forme réduite par l'hydrogène dans la déshydrogénation d'aminoalcools en acides amino-carboxyliques.

22. Utilisation du catalyseur fabriqué selon l'une ou plusieurs des revendications 11 à 20 sous la forme réduite par l'hydrogène dans la déshydrogénation (des dérivés) d'éthylène glycol en oxacides carboxyliques.
